# EUROPÄISCHE PATENTSCHRIFT

(11) **EP 0 663 407 B1**
(45) Veröffentlichungstag und Bekanntmachung des Hinweises auf die Patenterteilung: **03.07.2002**
(21) Anmeldenummer: 94810753.7
(22) Anmeldetag: 28.12.1994
(51) Int. Cl.: C07K 14/775

(54) **Verfahren zur Herstellung von rekonstituiertem Lipoprotein**
Process for preparation of reconstituted lipoproteins
Procédé pour la préparation du lipoprotéine reconstituée

(30) Priorität: 31.12.1993 EP 93810920
(43) Veröffentlichungstag der Anmeldung: 19.07.1995
(73) Patentinhaber: ZLB Bioplasma AG, 3014 Bern (CH)
(72) Erfinder: Lerch, Peter, Dr., CH-3007 Bern (CH); Hodler, Gerhard, CH-3076 Worb (CH); Förtsch, Vreni, CH-4600 Olten (CH)
(74) Vertreter: Fischer, Franz Josef

(56) Entgegenhaltungen:
- EP-A- 0 277 849
- EP-A- 0 329 605
- WO-A-88/09345
- WO-A-93/25581
- EXP LUNG RES, 1984, 6 (3-4) P255-70, UNITED STATES, JONAS A 'A review of plasma apolipoprotein A-I interactions with phosphatidylcholines.'
- JONAS A 'Reconstitution of high-density lipoproteins.' , METHODS ENZYMOL 128, 1986, P. 553-82, ED. BY J.P. SEGREST AND J.J. ALBERS, PUB. ACADEMIC PRESS , UNITED STATES * Seite 560 - Seite 565 *

## Beschreibung

Die vorliegende Erfindung bezieht sich auf ein Verfahren zur Herstellung von rekonstituierten Lipoproteinen (*reconstituted High Density Lipoproteins*; rHDL) aus Apolipoproteinen und Lipiden im industriellen Massstab. Die rHDL haben die Eigenschaft, dass sie Lipide und lipidähnliche Stoffe im Organismus binden, transportieren und deren Aktivitäten beeinflussen können. Die rHDL sind demzufolge nützlich für verschiedene prophylaktische und therapeutische Behandlungen von Krankheiten, die in einem Zusammenhang mit Lipiden und lipidähnlichen Stoffen stehen.

Die Lipoproteine des menschlichen Blutes können eine Reihe von unterschiedlichen Funktionen ausüben. Eine gut untersuchte und bekannte Funktion von Lipoproteinen ist der Transport von Lipiden. Lipoproteine haben die Fähigkeit, wasserunlösliche Lipide aufzunehmen, in wässerigem Milieu zu transportieren und an ihren Zielort zu bringen. Seit längerer Zeit werden die einzelnen Klassen der Lipoproteine im Zusammenhang mit Störungen des Lipidstoffwechsels näher untersucht. In den meisten westlichen Ländern konnte mittels epidemiologischer Studien eine positive Korrelation gezeigt werden zwischen Herz- Kreislauferkrankungen und hohem Plasmacholesterin oder hohem LDL-Cholesterin *(Low Density Lipoprotein -* Cholesterin), und eine negative Korrelation zu hohem HDL oder hohem HDL-Cholesterin. Obschon eine ganze Reihe von Medikamenten auf dem Markt erhältlich sind, welche eine Lipid-senkende Wirkung gezeigt haben, ist denkbar, dass in gewissen Situationen eine Substitution von geeigneten Lipoproteinen angezeigt ist. Für solche Fälle kommen in erster Linie die "guten" Lipoproteine wie die HDL in Frage, oder HDL-ähnliche Partikel, wie aus isolierten Apolipoproteinen und geeigneten Lipiden rekonstituierte HDL (rHDL).

Neben den Lipiden, welche durch die Nahrung aufgenommen werden, können von den Lipoproteinen auch andere Lipide oder lipidähnliche Stoffe transportiert oder gebunden werden. Beispielsweise können Bestandteile von abgestorbenen Zellen an Lipoproteine gebunden werden und einer neuen Funktion zugeführt werden. Es können aber auch lipidähnliche Stoffe an Lipoproteine gebunden werden, wie z.B. Lipopolysaccharid (LPS). Lipopolysaccharide oder Endotoxine sind Bestandteile von Membranen gramnegativer Bakterien. Falls genügend grosse Mengen von Endotoxin in den Kreislauf gelangen kann dies zu septischem Schock oder sogar zum Tode führen. Als Folge der Bindung von LPS an Lipoproteine kann die Funktion oder Aktivität des LPS moduliert werden. Die Aktivität von LPS kann in vivo und in vitro durch Zugabe von Lipoproteinen oder lipoproteinähnlichen Partikeln erheblich beeinflusst werden. So konnte gezeigt werden, dass in vivo die Zugabe von rekonstituiertem HDL die Bildung von *Tumornekrosisfaktor* (TNF), einem wichtigen Mediator der Sepsis, gehemmt werden kann. In vivo konnten somit durch Zugabe von HDL oder rHDL die Symptome des Schocks erheblich reduziert werden.

Neben Wechselwirkungen von Lipoproteinen oder rekonstituierten Lipoproteinen mit Lipiden oder lipidähnlichen Substanzen sind auch Wechselwirkungen von Lipoproteinen mit Proteinen beschrieben worden:
- Lipoproteine können mit einzelnen Komponenten des Komplementsystems Wechselwirkungen eingehen und dadurch deren Aktivität beeinflussen;
- ebenfalls sind Komponenten des Gerinnungssystems bekannt, welche in der Lipoproteinfraktion zu finden sind, das heisst, assoziiert sind mit bestimmten Lipoproteinen;
- Akutphasenproteine wie das Serum Amyloid A (SAA) sind in der HDL Fraktion gefunden worden;
- und weiter kann die Adsorption von gewissen Proteinen an Oberflächen durch Vorbehandlung mit Lipoproteinen beeinflusst werden.

Lipoproteine können aber auch durch spezifische oder unspezifische Bindung an Zellen deren Aktivität beeinflussen:
- Die Aktivierbarkeit von Plättchen kann durch Bindung von HDL reduziert oder durch Zugabe von LDL stimuliert werden;
- Monozyten und Makrophagen besitzen ebenfalls Rezeptoren für Lipoproteine; die Bindung oder Aufnahme von Lipoproteinen kann zu Änderungen der Aktivitäten dieser Zellen führen;
- Die Aktivität von weiteren Zellen des Immunsystems wie die Neutrophilen kann ebenfalls durch Bindung von Lipoproteinen modifiziert oder moduliert werden;
- Weiter kann auch das Zellwachstum von Tumorzellen, gezeigt am Beispiel der Glyoblastomazellen, durch Lipoproteine beeinflusst werden.

In der Literatur sind ferner Berichte zu finden, welche Wechselwirkungen von Lipoproteinen mit Pathogenen beschreiben, beispielsweise wird Lipoproteinen eine anti-mikrobielle Aktivität zugeschrieben. Es wurde gezeigt, dass Viren mittels Lipoproteinen inaktiviert werden können, oder am Beispiel von Trypanosomen konnten Parasiten beeinflusst bzw. gehemmt werden.

Diese Beispiele zeigen vielfältige Möglichkeiten für den Einsatz von Lipoproteinen oder rHDL für prophylaktische und therapeutische Anwendung.

Die Lipoproteine werden in vier Hauptklassen aufgeteilt: Chylomikronen, dies sind Partikel, welche vorwiegend aus Triglyceriden bestehen und normalerweise nur nach fetthaltigen Mahlzeiten in grösseren Mengen im Plasma auftauchen. VLDL, *Very Low Density Lipoproteins,* LDL, *Low Density Lipoproteins,* und HDL, *High Density Lipoproteins.* Die Nomenklatur ist abgeleitet von der Isolierung der Lipoproteine mittels Ultrazentrifugation. Mit dieser Methode werden die Lipoproteine auf klassische Art und Weise in einem Dichtegradienten isoliert. Diese Methode ist nur für den Labormassstab anwendbar, da sie zeitlich und apparativ sehr aufwendig ist. Bestenfalls können damit in einigen Tagen einige Hundert Milligramm Lipoproteine oder Apolipoproteine gewonnen werden. Andere Methoden zur Isolierung von Apolipoproteinen oder Lipoproteinen sind ebenfalls seit längerer Zeit bekannt; sie basieren vielfach auf der Fällung mittels divalenten Kationen und/oder beispielsweise Polyethylenglycol oder Dextran. Eine weitere Möglichkeit zur Isolierung von Apolipoproteinen ist die Fällung mittels Alkoholfraktionierung, wie dies im Patent Nr. EP 0 329 605 B1 beschrieben wurde. Mit dieser letzteren Methode ist es möglich, grössere Mengen von Apolipoprotein A-I (apoA-I) oder Fraktionen, welche angereichert sind an apoA-I, zu isolieren und für therapeutische Anwendungen zur Verfügung zu stellen. Mit so isoliertem apoA-I oder Proteinfraktionen angereichert an apoA-I wurden eine Reihe von Versuchen durchgeführt, sowohl in Tieren als auch an Menschen. Anhand dieser Versuche konnte einerseits die Sicherheit dieser Produkte bezüglich Viren gezeigt werden, aber auch dass apoA-I in der verwendeten Form zu keinerlei bedeutenden Nebenreaktionen an Mensch oder Tier führt. Allerdings konnte mit in vitro Versuchen keine der gewünschten Aktivitäten wie beispielsweise Cholesterin Transport oder Wirkung von apoA-I auf Zellen wie Neutrophile, Makrophagen, Monozyten oder Plättchen gefunden werden. Bei in vivo Versuchen wurden sowohl im Tier als auch im Menschen sehr kurze Halbwertszeiten von apoA-I im Plasma beobachtet. Aufgrund des Molekulargewichts von freiem apoA-I (28'000 Dalton) besteht die Möglichkeit, dass apoA-I durch die Niere ausgeschieden wird. Es konnte tatsächlich im Urin von Kaninchen apoA-I nachgewiesen werden. Diese Resultate bedeuten, dass so infundiertes apoA-I in grösseren Mengen nicht in die gewünschte Lipoproteinfraktion verteilt wird. Das apoA-I wird deshalb wegen seiner kurzen Halbwertszeit in vivo höchstens während einer sehr kurzen Zeit seine Wirkung ausüben können. Eine geeignetere Darreichungsform ist deshalb, das apoA-I in einem Lipoprotein oder lipoproteinähnlichem Partikel zu infundieren.

Methoden zur Herstellung von rekonstituierten Lipoproteinen sind in der Literatur beschrieben, insbesondere für die Apolipoproteine A-I, A-II, A-IV, apoC und apoE (A. Jonas, Methods in Enzymology **128**, 553-582 (1986)). Das häufigste Lipid, das für die Rekonstitution angewendet wird, ist Phosphatidylcholin, entweder aus Eiern oder aus Sojabohnen extrahiert. Andere Phospholipide werden ebenfalls eingesetzt, ebenso Lipide wie Triglyceride oder Cholesterin. Zur Rekonstitution werden die Lipide zuerst in einem organischen Lösungsmittel aufgelöst, welches anschliessend unter Stickstoff abgedampft wird. Bei diesem Vorgehen wird das Lipid in einem dünnen Film an eine Glaswand gebunden. Danach wird das Apolipoprotein und ein Detergens, normalerweise Natriumcholat, zugegeben und gemischt. Das zugegebene Natriumcholat bewirkt eine Dispersion der Lipide. Nach einer geeigneten Inkubationszeit wird die Mischung gegen grosse Mengen von Puffer während längerer Zeit dialysiert; dabei wird einerseits das Natriumcholat grösstenteils entfernt, und gleichzeitig lagern sich spontan Lipide und Apolipoproteine zu Lipoproteinen oder sogenannten rekonstituierten Lipoproteinen zusammen. Als Alternativen zur Dialyse stehen hydrophobe Adsorbentien zur Verfügung, welche spezifisch Detergentien adsorbieren können (Bio-Beads SM-2, Bio Rad; Amberlite XAD-2, Rohm & Haas) (E.A. Bonomo, J.B. Swaney, J. Lipid Res. **29**, 380-384 (1988)), oder die Abtrennung des Detergens mittels Gelchromatografie. Lipoproteine können auch ohne Detergenzien hergestellt werden, beispielsweise durch Inkubation einer wässerigen Suspension eines geeigneten Lipids mit Apolipoproteinen, Zugabe von Lipid, gelöst in einem organischen Lösungsmittel, zu Apolipoproteinen, mit oder ohne zusätzliche Erwärmung der Mischung, oder durch die Behandlung einer apoA-I - Lipid - Mischung mit Ultraschall. Mit diesen Methoden können ausgehend z.B. von apoA-I und Phosphatidylcholin scheibenförmige Partikel erhalten werden, welche nascierenden Lipoproteinen entsprechen. Anschliessend an die Inkubation werden gewöhnlich ungebundenes Apolipoprotein und freies Lipid mittels Zentrifugation oder Gelchromatografie abgetrennt, um die homogenen, rekonstituierten Lipoprotein Partikel zu isolieren.

In der US-A-5 128 318 ist ein Verfahren für die Herstellung von rekonstituiertem HDL beschrieben, worin Phosphatidylcholin mit Hilfe eines organischen Lösungsmittels in Lösung gebracht werden.

D1 (EP-A-0 325 605) beschäftigt sich mit einem Verfahren zur Herstellung von Apolipoproteinen aus Blut plasma- oder Serumfraktionen, WO-A-93 25581(D2) beschreibt ein Verfahren zur Herstellung eines Phospholipid-Protein komplexes.

Die oben beschriebenen Methoden zur Herstellung von rekonstituierten Lipoproteinen sind nur für kleinere Mengen von einigen Milligramm bis höchstens einigen Gramm im Labormassstab geeignet:
- hohe Verdünnungen der Lösungen in Zwischenprodukten verunmöglichen die Verarbeitung der Mischungen in der geforderten Zeit;
- die Infrastruktur für die grossen Volumen ist normalerweise nicht vorhanden;
- die verwendeten organischen Lösungsmittel sind nur bedingt umweltverträglich;
- die Endprodukte sind nicht lagerfähig;
- die Konzentration der Endprodukte ist zu gering, die in den Patienten zu infundierenden Volumen wären zu gross;
- die Produkte müssen normalerweise weiter gereinigt werden, beispielsweise mittels Gelchromatografie um ungebundenes Lipid oder ungebundenes Protein von den rHDL Partikeln abzutrennen..

Weiter ist in A. Hubsch et al., Circulatory Shock **40**, 14-23 (1993) ein Verfahren zur Herstellung von rekonstituierten Lipoproteinen beschrieben, worin ein Verhältnis Apolipoprotein zu Lipid von 1:200 verwendet wird. Dieses Vorgehen hat zur Folge, dass das erhaltene Produkt einen beträchtlichen Anteil an freiem Lipid aufweist, was seine therapeutische Anwendbarkeit ungünstig beeinflusst.

Für die therapeutische oder prophylaktische Anwendung von rHDL am Menschen sind pro Dosis rHDL Mengen im Gramm Bereich notwendig, um signifikante Steigerungen des apoA-I - oder HDL - Spiegels im Plasma zu erzielen. Eine wirtschaftliche Produktion von rHDL für klinische Anwendungen im kg- oder noch grösseren Massstab ist mit den oben beschriebenen Methoden nicht möglich.

Es ist demzufolge die Aufgabe der vorliegenden Erfindung ein Verfahren zur Herstellung von rHDL zur Verfügung zu stellen, welches frei von den oben angeführten Unzulänglichkeiten ist und dessen Produkt insbesondere keine grossen Anteile freies Lipid oder freies apoA-I enthält. Ein Ziel besteht darin, ein Verfahren zur Verfügung zu stellen, das ohne Zusatz organischer Lösungsmitteln durchgeführt werden kann. Es wurde gefunden, dass rekonstituierte Lipoproteine, insbesondere rekonstituierte HDL (rHDL), mit einfachen, schnellen und industriell anwendbaren Verfahren aus Apolipoproteinen und Lipiden hergestellt werden können.

Gegenstand der vorliegenden Erfindung ist demzufolge ein Verfahren zur industriellen Herstellung eines Präparates, das rekonstituierte Lipoproteinpartikel hoher Dichte (rHDL) aus Apolipoprotein A-I und Phosphtidylcholin enthält, und bei einem Proteingehalt von 20 ± 2 g/l und einer Temperatur von 20°C ± 2°C eine Trübung von weniger als 40 NTU (*Nephelometric Turbidity Unit*) aufweist, welches dadurch gekennzeichnet ist, dass
- eine Apolipoprotein A-I-Phosphatidylcholin-Detergens-Mischung hergestellt wird, indem (a) eine wässerige Apolipoprotein A-I-Lösung mit einer Konzentration von 1 - 40 g Protein/I mit (b) einer wässerigen Phosphatidylcholin-Detergens-Lösung, ohne Verwendung von organischen Lösungsmitteln, gemischt wird, wobei das molare Verhältnis Phosphatidylcholin zu Detergens im Bereich von 1:0.5 bis 1:4.0 liegt und das Gewichtsverhältnis Apolipoprotein A-I zu Phosphatidylcholin im Bereich von 1:1.5 bis 1:5.0 liegt,
- die erhaltene Apolipoprotein A-I-Phosphatidylcholin-Detergens-Mischung anschliessend bei einer Temperatur im Bereich der Phasenumwandlungstemperatur ± 10°C des Phosphatidylcholins in Wasser inkubiert wird,
- das Detergens durch Ausschluss nach Grösse oder Adsorption an ein Adsorbens soweit abgetrennt wird, dass sich Apolipoprotein A-I-Phosphatidylcholin-Partikel mit einem Durchmesser von 5 - 50 nm mit einer Masse von 50'000 bis 1'000'000 Dalton bilden,
- wobei ohne weitere Reinigungsschritte ein Präparat, das rekonstituierte Lipoproteinpartikel hoher Dichte (rHDL) aus Apolipoprotein A-I und Phosphtidylcholin enthält, erhalten wird, in welchem sowohl mehr als 95% des eingesetzten Apolipoproteins A-I als auch mehr als 90% des gesamten Phosphatidylcholins gebunden sind.

Gegenstand der vorliegenden Erfindung ist ebenfalls ein Verfahren zur industriellen Herstellung eines stabilen Lyophilisates, das rekonstituierte Lipoproteinpartikel hoher Dichte (rHDL) aus Apolipoprotein A-I und Phosphtidylcholin enthält, das dadurch gekennzeichnet ist, dass
- eine Apolipoprotein A-I-Phosphatidylcholin-Detergens-Mischung hergestellt wird, indem (a) eine wässerige Apolipoprotein A-I-Lösung mit einer Konzentration von 1 - 40 g Protein/l mit (b) einer wässerigen Phosphatidylcholin-Detergens-Lösung, ohne Verwendung von organischen Lösungsmitteln, gemischt wird, wobei das molare Verhältnis Phosphatidylcholin zu Detergens im Bereich von 1:0.5 bis 1:4.0 liegt und das Gewichtsverhältnis Apolipoprotein A-I zu Phosphatidylcholin im Bereich von 1:1.5 bis 1:5.0 liegt,
- die erhaltene Apolipoprotein A-I-Phosphatidylcholin-Detergens-Mischung anschliessend bei einer Temperatur im Bereich der Phasenumwandlungstemperatur ± 10°C des Phosphatidylcholins in Wasser inkubiert wird,
- das Detergens durch Ausschluss nach Grösse oder Adsorption an ein Adsorbens soweit abgetrennt wird, dass sich Apolipoprotein A-I-Phosphatidylcholin-Partikel mit einem Durchmesser von 5 - 50 nm mit einer Masse von 50'000 bis 1'000'000 Dalton bilden,
- wobei ohne weitere Reinigungsschritte ein Präparat, das rekonstituierte Lipoproteinpartikel hoher Dichte (rHDL) aus Apolipoprotein A-I und Phosphtidylcholin enthält, erhalten wird, in welchem sowohl mehr als 95% des eingesetzten Apolipoprotein A-I als auch mehr als 90% des gesamten Phosphatidylcholins gebunden sind, in Form eines flüssigen Produktes, das bei einem Proteingehalt von 20 ± 2 g/l und einer Temperatur von 20°C ± 2°C eine Trübung von weniger als 40 NTU aufweist, und das enthaltene Produkt durch Lyophilisation in Gegenwart eines Stabilisators ausgewählt aus der Gruppe der Kohlenhydrate stabilisiert wird.

Unter die verwendeten Apolipoproteine fallen auch rekombinante Apolipoproteine, Apolipoproteine aus einer angereicherten Fraktion von Apolipoprotein A-I aus humanem Plasma, Fragmente von Apolipoproteinen oder natürliche, synthetische oder rekombinante Polypeptide mit amphipatischen Eigenschaften. Die Fragmente von Apolipoproteinen sind erhältlich durch chemische oder enzymatische Fragmentierung von natürlichen oder synthetischen Apolipoproteinen. Als Beispiel für eine chemische Fragmentierung wird die Behandlung mit Bromcyan erwähnt. Als Beispiele für die enzymatische Spaltung werden Trypsin oder Chymotrypsin aufgeführt.

Die erfindungsgemäss eingesetzte Lipid-Detergens-Lösung enthält als Lipid beispielsweise Phospholipid, das aus Sojabohnen oder Eiern stammen kann, Cholesterin, Cholesterin-Ester, Fettsäuren oder Triglyceride. Die Detergentien sind vorzugsweise Gallensäuren oder Salze davon z.B. Cholsäure-Natriumsalz oder Natrium-Desoxycholsäure. Zur Solubilisierung der Lipide sind dabei keine organischen Lösungmittel erforderlich.

Der in der vorliegenden Beschreibung angegebene Temperaturwert von 20 ± 2°C umfasst alle Werte, die in den Bereich 18 °C bis 22 °C fallen. Der Proteingehalt von 20 ± 2 g/l umfasst alle Konzentrationen im Bereich von 18 g/l bis 22 g/l. Die Angabe "Phasenumwandlungstemperatur ± 10 °C" umfasst alle Temperaturwerte, die in den Bereich fallen, der zwischen der Temperatur, die um 10 °C tiefer ist als die Phasenumwandlungstemperatur des entsprechenden Lipids in wässerigem Milieu, und derjenigen, die 10 °C über diesem stoffspezifischen Wert liegt. Diese Werte liegen zwischen -5 °C und 50 °C.

Bevorzugtes Ausgangsmaterial sind Lipoproteine, die aus humanem Plasma stammen, welche mittels geeigneten Methoden vireninaktiviert, wie z.B. pasteurisiert, sind. Die bei diesem Vorgang entstehenden denaturierten Proteine (Aggregate) werden anschliessend durch eine Inkubation bei leicht alkalischem pH und leicht erhöhter Temperatur in Gegenwart einer chaotropen Komponente, wie z.B. Harnstoff oder Guanidin-Hydrochlorid, renaturiert, so dass nach Umpuffern des Proteins in 10 mM NaCI >70% des apoA-I in monomerer Form vorliegt (Bestimmung mittels analytischer *Size Exclusion Chromatography*: 40 µg apoA-I wurde aufgetragen auf eine TSK G3000SW Ultropac Säule (LKB), in 10 mM Natriumphosphat, 0.02% Natriumazid, pH 7.4, mit einem Fluss von 0.4 ml/min; Messung des Eluats bei 280 nm).

Im weiteren Verfahren wird Apolipoprotein A-I in hoher Konzentration mit einer Lösung von Phosphatidylcholin in Gallensäuren oder deren Salze (z.B. Cholat, Desoxycholat, Ursodesoxycholat) gemischt, wobei auf den Einsatz von organischen Lösungsmitteln verzichtet werden kann. Im Gegensatz zu Arbeiten beschrieben von Hubsch et al. (Circulatory Shock **40**, 14-23 (1993)), wird das Verhältnis von Apolipoprotein zu Lipid so gewählt, dass sich im Endprodukt weder grössere Mengen von freiem Lipid noch von freiem Apolipoprotein finden, so dass auf eine weitere Reinigung der rHDL verzichtet werden kann. Insbesondere wird das apoA-I: PC Verhältnis reduziert, das wesentlich unter 1 : 200 (M : M; Gewichtsverhältnis ca. 1 : 5.5) liegt, nämlich auf ein Verhältnis im Bereich von 1 : 50 bis 1 : 180, und vorzugsweise von 1 : 100 bis 1 : 150 (Molverhältnis; entspricht einem Gewichtsverhältnis von 1 : 2.8 bis 1 : 4.2 für apoA-l und PC aus Soja). Dies, kombiniert mit einer Diafiltrationsmethode, führt zu einem Produkt mit einem geringeren Gehalt an freien Lipiden (quantifiziert mittels *Size Exclusion Chromatography;* Gelfiltration mittels Superose 6; s. unten), und gleichzeitig zu einer erheblich tieferen Konzentration von Gallensäuren im Endprodukt; beides, hohe Gallensäure-Konzentrationen und hoher Gehalt an freiem PC, können *in vitro* und *in vivo* zur Schädigung von Zellen führen, und müssen deshalb genau kontrolliert werden. Die Cholatkonzentration wird einerseits optimiert auf das Verhältnis apoA-I : PC und allenfalls gleichzeitig abgestimmt auf weitere Lipidzusätze, insbesondere Cholesterin. Auch hier wurde die optimale Konzentration bestimmt durch einen möglichst kleinen Anteil freien Lipids und freien apoA-I im Endprodukt; für ein rHDL Präparat mit einem apoA-I : PC Verhältnis von 1 : 150 wird ein molares Verhältnis von Na-Cholat von 1: 200 gefunden (d.h. apoA-I: PC: Na-Cholat = 1 : 150 : 200 (M: M : M), während der nachfolgend beschriebenen Inkubation). Nach einer Inkubation von 4 - 16 h bei 0°C - 2°C (für PC aus Soja) wird die Konzentration der Gallensäure mittels Diaflitration gesenkt, mit einer Ultrafiltrationsmembran von Porengrössen für globuläre Proteine zwischen 1'000 und 100'000 Dalton, bevorzugt unter 30'000 Dalton. Der dazu benötigte Puffer weist eine tiefe lonenstärke von unter 100 mmol/l auf, vorzugsweise unter 10 mmol/l, bei einem pH über 6, vorzugsweise 7.5 - 9, und einen Zuckergehalt von beispielsweise 1% Saccharose; unter diesen Bedingungen genügen 1 bis maximal 2 I Puffer pro Gramm eingesetztes Protein, um einerseits die nötige tiefe Detergenskonzentration, und andererseits die gewünschte Partikelgrösse Verteilung zu erreichen; diese Puffervolumina sind um ein Vielfaches kleiner (10 - 200 x) als in früher beschriebenen Methoden. Falls nötig, wird die Detergenskonzentration mit einem zusätzlichen Adsorptionsschritt mit Amberlite auf eine gewünschte Konzentration eingestellt. Mit der oben erwähnten Technologie der Diafiltration wird das Produkt auf eine hohe Konzentration von 10 - 50 g Protein/l eingestellt, und anschliessend in Gegenwart eines Stabilisators wie Saccharose zu einem stabilen, lagerfähigen Endprodukt verarbeitet (flüssig oder lyophilisiert). Das Lyophilisat wird vor Anwendung mit Wasser aufgelöst, wobei eine klare, allenfalls leicht opaleszente Lösung erhalten wird, welche je nach Lipidgehalt leicht gelblich gefärbt ist. In dieser Lösung konnten die nach der Herstellung gemessenen rHDL Partikel (Disks, Scheibchen) in praktisch unveränderter Form wieder nachgewiesen werden; mittels *Size Exclusion Chromatography* wird ein Anteil von < 10% Aggregaten (grösstenteils freies Lipid) und ein Anteil von < 5% von freiem Apolipoprotein gefunden. Die im flüssigen Endprodukt vor oder nach Lyophilisation bestimmte Trübung liegt bei einer Proteinkonzentration von 20 g/l unter 40 NTU (*Nephelometric Turbidity Unit*). Der mit einem enzymatische Farbtest gemessene Cholatgehalt ist kleiner als 0.5 g Cholsäure-Natriumsalz/g Protein (Die Gallensäurebestimmung erfolgt über die Bildung von NADH in Gegenwart von NAD+ mit Hilfe der 3-α-Hydroxysteroid-dehydrogenase; das gebildete NADH reagiert mit Nitrotetrazolium-Blau unter der katalytischen Wirkung von Diaphorase zu einem blauen Formazan-Derivat, welches photometrisch bestimmt wird.).

Die lyophilisierten rHDL liegen bei Betrachtung im Elektronenmikroskop nach Auflösen in einem geeignetem Volumen Lösungsmittel, vorzugsweise Wasser, als scheibenförmige Partikel, analog zu nascierenden HDL vor, die einen Durchmesser von 5 - 50 nm normalerweise8 - 20 nm und eine Dicke von 2 - 6 nm aufweisen. Mit Analysemethoden zur Bestimmung von Partikelgrössen und deren relative Verteilung, beispielsweise durch Gelfiltration (*Size Exclusion Chromatography)* in einem physiologischen Puffer mit einer Superose® 6 HR 10/30 Säule (Pharmacia Biotech) liegen mehr als 80% der Partikel im Molekulargewichtsbereich von 100'000 Dalton bis 1'000'000 Dalton. Ebenfalls bei mehr als 80% der Partikel liegt auf Grund einer Gradienten-Gel-Elektrophorese eine Molekulargewichtsverteilung im Bereich von 50'000 bis 1'000'000 Dalton vor (Methode nach A.V. Nichols et al., Methods in Enzymology 128, 417-431 (1986)).

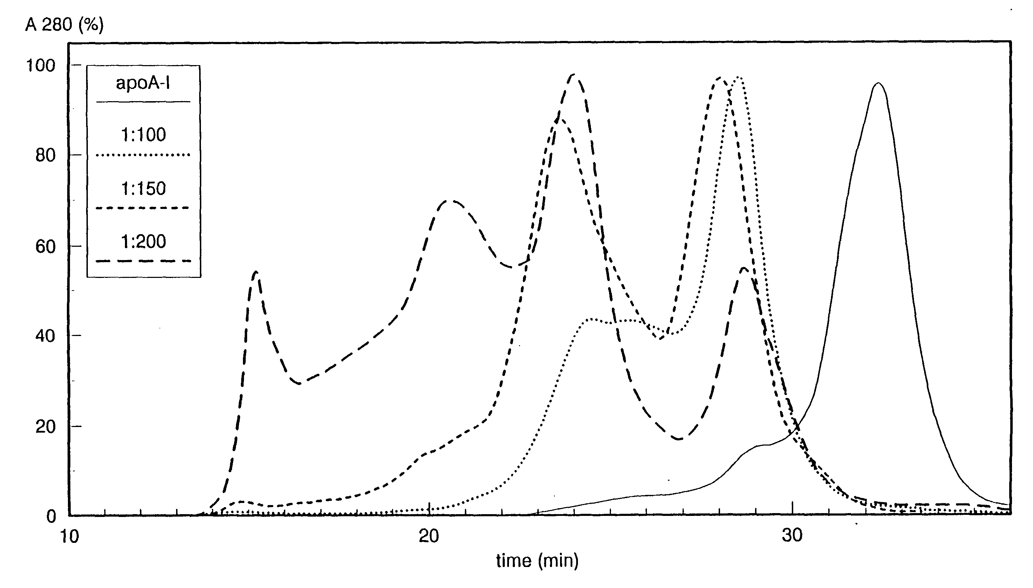

Die einzige Figur dient zum besseren Verständnis der Erfindung und zur Stützung der obigen Ausführungen; sie zeigt ein Elutionsdiagramm (Absorption-Elutionszeit Diagramm) von Gelfiltrationen von rHDL Partikeln, die durch den Einsatz von verschiedenen Verhältnissen von apoA-I zu Phosphatidylcholin (Apolipoprotein zu Lipid-Verhältnissen) hergestellt wurden. *[High Performance Size Exclusion Chromatography* von apoA-I und rHDL Partikeln; Auftrennung von 200 µg rHDL in 100 µl 0.9% NaCI auf einer Superose® 6 HR 10/30 Säule (Pharmacia Biotech) in PBS (10 mM Natriumphosphat, 150 mM NaCI, pH 7.4) mit einem Fluss von 0.5 ml/min]. Die Absorption des Säulen-Eluats wurde gemessen bei 280 nm (L.L. Rudel, C.A. Marzetta and F.L. Johnson, Methods in Enzymology **129**, 45-57 (1986)); zur Bestimmung des Gehaltes einzelner Fraktionen im Chromatogramm wird die Fläche unter der Kurve berechnet. Bei einer Elutionskurve des 1 : 200 - Produktes ist ersichtlich, dass zu Beginn der Elution freie Lipide ausgewaschen werden, während dies bei den 1 : 100 - und 1 : 150 - Produkten nicht der Fall ist. Zum Vergleich ist ebenfalls die Kurve des reinen Apolipoproteins (apoA-I) angegeben.

Die nachstehenden Beispiele dienen der Erläuterung der vorliegenden Erfindung, wobei diese nicht als Einschränkung der Erfindungsdefinition zu verstehen sind.

### Beispiel 1:

Ein kg apoA-I wurde gelöst in 500 I 0.15 mol/l NaCI. Die Lipidlösung wurde separat folgendermassen hergestellt: Phosphatidylcholin aus Sojabohnenöl (Phospholipon 90®, Nattermann, Köln) wurde gelöst in einem Puffer bestehend aus 10 mmol/l Tris/HCl, 150 mmol/l NaCI, 1 mmol/l EDTA, pH 8.0. 2.8 kg Phosphatidylcholin und 2.325 kg Cholsäure Natriumsalz wurden in diesem Puffer ad 100 I gelöst. Es wurde 1 - 2 Stunden gerührt, und, falls nötig, bis ca. 40°C aufgewärmt, bis die Lösung klar war. Anschliessend wurde abgekühlt auf 4°C und 100 I dieser Lipid-Cholat-Lösung wurden gemischt mit 1 kg apoA-I in 500 l. Das Gemisch wurde über Nacht bei 2 - 4°C langsam gerührt. Nach dieser Inkubation wurde steril filtriert und bei 4°C diafiltriert mit einem Pellicon mit PTGC-Cassetten, *nominal molecular weight limit* (NMWL) = 10'000 Dalton: zuerst mit 4 Volumen 5 mmol/l Natriumhydrogencarbonat und anschliessend 2 Volumen 10% Saccharose, wobei das Volumen des Produktes konstant gehalten wurde. Die Konzentration wurde anschliessend langsam erhöht, bis eine Proteinkonzentration von 20 g/l erreicht war. Diese Lösung wurde wiederum steril filtriert und in Flaschen abgefüllt und anschliessend lyophilisiert. Während des ganzen Vorgangs wurde darauf geachtet, dass insbesondere die Lipidlösung geschützt war vor Luft, Licht und allzu hohen Temperaturen. Das Endprodukt aufgelöst in der geeigneten Menge Wasser welche eine Proteinkonzentration von 20 ± 2 g/l ergab, wies ein molares Verhältnis von apoA-I zu Phosphatidylcholin von 1:100 auf (Mol: Mol) auf, mit weniger als 5% freiem Lipid, ohne messbare Anteile von freiem Protein und einer Trübung von kleiner als 40 NTU.

### Beispiel 2:

Zehn kg apoA-I wurden in 2000 I10 mmol/l NaCI bereitgestellt. 1.38 kg Cholesterin und 29.9 kg Cholsäure Natriumsalz wurden in 200 I eines Puffers enthaltend 10 mmol/l Tris-HCl, 150 mmol/l Kochsalz 1 mmol/l EDTA, pH 8.0 gerührt. Die Temperatur wurde erhöht bis auf 65°C und die Mischung zwei Stunden gerührt, bis die Lösung klar war. Anschliessend wurde abgekühlt auf 20°C und 27.9 kg Phosphatidylcholin wurden zugegeben. Die Lösung wurde auf 4°C abgekühlt und 2 Stunden gerührt. Diese Mischung wurde zur Proteinlösung gegeben, es wurde gemischt, und anschliessend steril filtriert. Das Filtrat wurde über Nacht (mindestens 16 Std.) bei 4°C langsam gerührt. Anschliessend folgte eine Diafiltration mit 4 Volumen 50 mmol/l NaCI, 1 mmol/l EDTA pH 7.5 während 2 - 4 Stunden. Danach wurde weiter diafiltriert mit weiteren 2 Volumen 10 % Saccharose. Diese Lösung wurde anschliessend auf 20 g/l Proteinkonzentration aufkonzentriert, steril filtriert, abgefüllt in Glasflaschen, und lyophilisiert. Die Flaschen wurden unter Vakuum verschlossen und bei 4°C im Dunkeln aufbewahrt. Mit dieser Methode wurden rHDL in einem molaren Verhältnis von apoA-I zu Phosphatidylcholin zu Cholesterin von 1:100:10 erhalten.

### Beispiel 3:

Herstellung eines rHDL mit einem Verhältnis von 1:150 apoA-I zu Phosphatidylcholin: 3.08 kg Natriumcholat wurden gelöst in 25 1 eines Puffers, 10 mmol/l Tris HCI, 10 mmol/l NaCI, 1 mmol/l EDTA, pH 8.0. Darin wurden weiter 4.2 kg Phosphatidylcholin während 2 Stunden bei Raumtemperatur gelöst. Anschliessend wurde 1 kg apoA-I in 200 110 mmol/l NaCI zugegeben und die Mischung über Nacht bei 0 - 4°C inkubiert. Anschliessend wurde bei konstantem Volumen des Produktes diafiltriert gegen 4 Volumen 50 mmol/l NaCI, 1 mmol/l EDTA pH 7.5, während 4 Stunden. Weiter wurde diafiltriert gegen 2 Volumen 1 % Saccharose. Schliesslich wurde aufkonzentriert auf 20 g/l Protein. Die Saccharosekonzentration wurde durch Zugabe von fester Saccharose auf 10% erhöht. Die Lösung wurde steril filtriert und lyophilisiert.

### Beispiel 4:

Herstellung eines rHDL mit einem apoA-I zu Phosphatidylcholin zu Cholesterin, Verhältnis von 1:100:10: 4.61 kg Natriumcholat wurden gelöst in 25 I eines Puffers enthaltend 10 mmol/l Tris-HCl, 10 mmol/l NaCI, 1 mmol/l EDTA, pH 8.0. 138 g Cholesterin wurden bei 65°C 2 Stunden in diesem Puffer Cholat Gemisch gelöst. Anschliessend wurde abgekühlt auf Raumtemperatur und 2.8 kg Phosphatidylcholin wurden während 1 Stunde darin gelöst. 1 kg apoA-I in 200 I 10 mmol/l NaCI wurden zugegeben. Es wurde inkubiert wie bei dem vorherigen Beispiel ebenfalls diafiltriert und aufkonzentriert wie oben.

### Beispiel 5:

ApoA-I zu PC zu Cholesterin 1:150:10: 5.38 kg Natriumcholat wurden gelöst Puffer wie in Beispiel 3 und 4 aufgeführt. 180 g Cholesterin darin bei 65°C während 2 Stunden gelöst. Anschliessend wurden 4.2 kg PC zugegeben und bei Raumtemperatur eine Stunde gelöst. Die Mischung wurde zugegeben zu 200 I apoA-I Lösung, 5 g pro Liter in 10 mmol/l NaCI, und über Nacht bei 4°C inkubiert. Diafiltration und Herstellung des Endproduktes wie oben.

### Beispiel 6:

Herstellung eines rHDL mit tiefem Natriumcholatgehalt. Die rHDL wurden hergestellt wie in den Beispielen 1 - 5 aufgeführt. Nach der Diafiltration und dem Aufkonzentrieren wurde ein Volumen rHDL Lösung gemischt mit Amberlite XAD-2 (2 Volumen) und diese Mischung wurde eine Stunde bei 4°C mit vorsichtigem Bewegen inkubiert. Nach der Inkubation wurde das rHDL abfiltriert, steril filtriert und lyophilisiert wie in den Beispielen 1 - 5 dargestellt.

### Beispiel 7:

400 g apoA-I in 80 ml 10 mmol/l NaCI wurden gemischt mit einer Lipidlösung bestehend aus 1.66 kg Phosphatidylcholin, 1.23 kg Natriumcholat in einem Puffer wie unter Beispiel 3 - 6 beschrieben. Die Mischung wurde 16 Stunden bei 0 - 2°C inkubiert. Anschliessend wurde diafiltriert mit 4 Volumen EDTA (0.1 mmol/l) und anschliessend 2 - 4 Volumen Saccharose (1%) und schliesslich aufkonzentriert auf 21 - 25 g/l Proteinkonzentration. Die rHDL-Lösung wurde danach eingestellt auf eine Endkonzentration von 10 % Saccharose und 20 g/l Protein. Es wurde sterilfiltriert und abgefüllt in Portionen von 50 g (1 g rHDL in 100 ml Flaschen) und lyophilisiert.

### Beispiel 8:

Aus 980 kg Vorfällung IV (Kistler, P., Nitschmann, H.; Vox Sang. 7, 414-424 (1962)) wurden durch ethanolische Fällung 11.2 kg Niederschlag apoA-I gewonnen. Dieser wurde in dreifacher Menge 4 molarer Guanidin-Hydrochlorid Lösung suspendiert. Das pH wurde auf 5.2 gestellt und während 10 Stunden bei 60°C pasteurisiert. Bei pH 7.5 und 45°C wurde das Protein solubilisiert. Anschliessend an eine Klärfiltration erfolgte mit einer 10 mM Kochsalzlösung ein Pufferwechsel mittels einer Gelfiltration (Sephadex G25, Pharmacia Biotech). Es wurden 160 kg apoA-I Lösung mit insgesamt 1040 g apoA-I erhalten. Die Proteinlösung wurde mit einer Lipidlösung während 2 bis 16 Stunden bei 0 bis 2°C inkubiert. Die Lipidlösung wurde separat bei Raumtemperatur hergestellt: Phosphatidylcholin aus Sojabohnenöl wurde gelöst in einem Puffer bestehend aus 10 mmol/l Tris-HCl, 10 mmol/l NaCI, 1 mmol/l EDTA, pH 8.0. In 26 kg dieses Puffers wurden 3203 g Natriumcholat und 4460 g Phosphatidylcholin gelöst. Bei der folgenden Diafiltration (NMWL = 10'000 Dalton) wurde zuerst das Protein-Lipid-Gemisch auf einen Proteingehalt von 7 g/l aufkonzentriert. Dann wurde gegen eine 1%-ige Saccharoselösung diafiltriert bis ein Cholatgehalt von weniger als 4 g/l erreicht wurde. Das pH wurde dabei immer auf mindestens 7.5 gehalten. Zuletzt wurde die Lösung auf 25 g/l Protein aufkonzentriert und mit Saccharose stabilisiert. Das Endprodukt enthielt 20 g/l apoA-I und 100 g/l Saccharose. In der *Size Exclusion Chromatography* wurden weniger als 5% freies Lipid und weniger als 1% freies apoA-I gefunden. Das Protein-Lipid-Gemisch wurde sterilfiltriert, zu je 50 ml abgefüllt und lyophilisiert. Das Endprodukt aufgelöst in der geeigneten Menge Wasser wies ein molares Verhältnis von apoA-I: Phosphatidylcholin von 1 : 140 [Mol: Mol] auf, und ein Gehalt an Cholsäure Natriumsalz von 0.25 g/g Protein.

## Patentansprüche

1. Verfahren zur industriellen Herstellung eines Präparates, das rekonstituierte Lipoproteinpartikel hoher Dichte (rHDL) aus Apolipoprotein A-I und Phosphatidylcholin enthält, und bei einem Proteingehalt von 20 ± 2 g/l und einer Temperatur von 20°C ± 2°C eine Trübung von weniger als 40 NTU aufweist, **dadurch gekennzeichnet, dass**
- eine Apolipoprotein A-l-Phosphatidylcholin-Detergens-Mischung hergestellt wird, indem (a) eine wässerige Apolipoprotein A-I-Lösung mit einer Konzentration von 1 - 40 g Protein/l mit (b) einer wässerigen Phosphatidylcholin-Detergens-Lösung, ohne Verwendung von organischen Lösungsmitteln, gemischt wird, wobei das molare Verhältnis Phosphatidylcholin zu Detergens im Bereich von 1:0.5 bis 1:4.0 liegt und das Gewichtsverhältnis Apolipoprotein A-I zu Phosphatidylcholin im Bereich von 1:1.5 bis 1:5.0 liegt,
- die erhaltene Apolipoprotein A-l-Phosphatidylcholin-Detergens-Mischung anschliessend bei einer Temperatur im Bereich der Phasenumwandlungstemperatur ± 10°C des Phosphatidylcholins in Wasser inkubiert wird,
- das Detergens durch Ausschluss nach Grösse oder Adsorption an ein Adsorbens soweit abgetrennt wird, dass sich Apolipoprotein A-I-Phosphatidylcholin-Partikel mit einem Durchmesser von 5 - 50 nm mit einer Masse von 50'000 bis 1'000'000 Dalton bilden,
- wobei ohne weitere Reinigungsschritte ein Präparat, das rekonstituierte Lipoproteinpartikel hoher Dichte (rHDL) aus Apolipoprotein A-I und Phosphatidylcholin enthält, erhalten wird, in welchem sowohl mehr als 95% des eingesetzten Apolipoprotein A-I als auch mehr als 90% des gesamten Phosphatidylcholins gebunden sind.

2. Verfahren zur industriellen Herstellung eines stabilen Lyophilisat-Präparates, das rekonstituierte Lipoproteinpartikel hoher Dichte (rHDL) aus Apolipoprotein A-I und Phosphatidylcholin enthält, **dadurch gekennzeichnet, dass** - eine Apolipoprotein A-I-Phosphatidylcholin-Detergens-Mischung hergestellt wird, indem (a) eine wässerige Apolipoprotein A-I-Lösung mit einer Konzentration von 1 - 40 g Protein/l mit (b) einer wässerigen Phosphatidylcholin-Detergens-Lösung, ohne Verwendung von organischen Lösungsmitteln, gemischt wird, wobei das molare Verhältnis Phosphatidylcholin zu Detergens im Bereich von 1:0.5 bis 1:4.0 liegt und das Gewichtsverhältnis Apolipoprotein A-l zu Phosphatidylcholin im Bereich von 1:1.5 bis 1:5.0 liegt,
-- die erhaltene Apolipoprotein A-I-Phosphatidylcholin-Detergens-Mischung anschliessend bei einer Temperatur im Bereich der Phasenumwandlungstemperatur ± 10°C des Phosphatidylcholins in Wasser inkubiert wird,
-- das Detergens durch Ausschluss nach Grösse oder Adsorption an ein Adsorbens soweit abgetrennt wird, dass sich Apolipoprotein A-I-Phosphatidylcholin-Partikel mit einem Durchmesser von 5 - 50 nm mit einer Masse von 50'000 bis 1'000'000 Dalton bilden,
-- wobei ohne weitere Reinigungsschritte ein Präparat, das rekonstituierte Lipoproteinpartikel hoher Dichte (rHDL) aus Apolipoprotein A-I und Phosphatidylcholin enthält, erhalten wird, in welchem sowohl mehr als 95% des eingesetzten Apolipoprotein A-I als auch mehr als 90% des gesamten Phosphatidylcholins gebunden sind, in Form eines flüssigen Produktes, das bei einem Proteingehalt von 20 ± 2 g/l und einer Temperatur von 20°C ± 2°C eine Trübung von weniger als 40 NTU aufweist und das enthaltene Produkt durch Lyophilisation in Gegenwart eines Stabilisators, ausgewählt aus der Gruppe der Kohlenhydrate, stabilisiert wird.

3. Verfahren nach Anspruch 1 oder 2, **dadurch gekennzeichnet, dass** das Abtrennen des Detergens durch Ausschluss nach Grösse durch Ultrafiltration erfolgt, wobei höchstens 2 I Puffer pro g Protein verwendet werden.

4. Verfahren nach einem der Ansprüche 1 bis 3, **dadurch gekennzeichnet, dass** nach Inkubation des Lipid-Apolipoprotein-Detergens-Gemisches das Detergens mittels Adsorption an ein hydrophobes Adsorbens entweder durch Batch Adsorption oder durch Säulenverfahren gebunden wird.

5. Verfahren nach Anspruch 4, **dadurch gekennzeichnet**, das das hydrophobe Adsorbens ein unlösliches vernetztes Polystyrol-Copolymer ist.

6. Verfahren nach einem der Ansprüche 1 bis 5, **dadurch gekennzeichnet, dass** die Apolipoprotein A-I-Phosphatidylcholin-Detergens-Lösung Phospholipide, Cholesterin, Cholesterin-Ester, Fettsäuren und/oder Triglyceride enthält.

7. Verfahren nach einem der Ansprüche 1 bis 6, **dadurch gekennzeichnet, dass** als Apolipoprotein A-I eine aus humanem Plasma an Apolipoprotein A-I angereicherte Fraktion eingesetzt wird, welche durch mindestens einen Vireninaktivierungsschritt, mittels Inkubation in einer wässerigen Lösung eines chaotropen Stoffes und einer anschliessenden Umpufferung in eine Lösung mit einer lonenstärke von weniger als 50 mmol/l, behandelt wurde, wonach mehr als 70 % des Lipoproteines in monomerer Form vorliegen.

8. Verfahren nach einem der Ansprüche 1 bis 7, **dadurch gekennzeichnet, dass** das Phosphatidylcholin synthetisches Phosphatidylcholin ist.

9. Verfahren nach einem der Ansprüche 1 bis 8, **dadurch gekennzeichnet, dass** das Phosphatidylcholin ein natürliches Phosphatidylcholin, vorzugsweise ein aus Sojabohnen oder Eiern extrahiertes Phosphatidylcholin ist.

10. Verfahren nach Anspruch 9, **dadurch gekennzeichnet, dass** Soja-Phosphatidylcholin verwendet wird, und die Inkubation der Apolipoprotein A-I- Phosphatidylcholin-Detergens Mischung bei einer Temperatur von 0 - 15°C erfolgt.

11. Verfahren nach einem der Ansprüche 1 bis 10, **dadurch gekennzeichnet, dass** das eingesetzte Detergens aus der Klasse der Gallensäuren oder einem Salz davon ausgewählt ist.

12. Verfahren nach Anspruch 11, **dadurch gekennzeichnet, dass** das Detergens Cholsäure-Natriumsalz oder Natrium-Desoxycholsäure ist.

13. Verfahren nach einem der Ansprüche 1 bis 12, **dadurch gekennzeichnet, dass** die wässerige Apolipoprotein A-I-Lösung rekombinante Apolipoproteine, Apolipoproteine aus einer angereicherten Fraktion von Apolipoprotein A-I aus humanem Plasma, Fragmente von Apolipoproteinen oder geeignete natürliche, synthetische oder rekombinante Polypeptide mit amphipatischen Eigenschaften enthält.

14. Verfahren nach einem der Ansprüche 1 bis 13, **dadurch gekennzeichnet, dass** nach der Inkubation des Apolipoprotein A-I-Phosphatidylcholin-Detergens-Gemisches der Gehalt des Detergens mittels Dialyse oder Diafiltration auf eine Konzentration von unter 0.5 g Detergens pro g Protein gesenkt wird.

15. Verfahren nach einem der Ansprüche 1 bis 14, **dadurch gekennzeichnet, dass** vor oder nach der Abtrennung des Detergens die Proteinkonzentration mittels Diafiltration auf 10 - 50 g/l erhöht wird.

16. Verfahren nach einem der Ansprüche 1 bis 15, **dadurch gekennzeichnet, dass** die wässerige Apolipoprotein A-I-Lösung oder die Lipid-Detergens-Lösung bei einem pH-Wert im Bereich von pH 6 bis pH 9 gepuffert ist.

17. Verfahren nach einem der Ansprüche 1 bis 16, **dadurch gekennzeichnet, dass** die wässerige Apolipoprotein-Lösung oder die Phosphatidylcholin-Detergens-Lösung bei einem pH-Wert im Bereich von pH 7.5 - 8.5 gepuffert ist

18. Verfahren nach einem der Ansprüche 2 bis 17, **dadurch gekennzeichnet, dass** das Produkt auf eine Proteinkonzentration von 5 - 50 g/l eingestellt wird und die Lyophilisation der Lösung zur Stabilisierung des Produktes in Gegenwart von 5-15% eines Disaccharides wie Saccharose oder von 2-10% eines Monosaccharides wie Mannit erfolgt.

## Claims

1. Method for industrially producing a preparation which contains reconstituted high density lipoprotein particles (rHDL) from apolipoprotein A-I and phosphatidyl choline, and which, at a protein content of 20 ± 2 g/l and at a temperature of 20 °C ± 2 °C, has a turbidity of less than 40 NTU, **characterised in that**
-- an apolipoprotein A-I- phosphatidyl choline- detergent mixture is prepared by mixing (a) an aqueous apolipoprotein A-I solution with a concentration of 1-40 g protein/I with (b) an aqueous phosphatidyl choline-detergent solution, without using organic solvents, the molar ratio of phosphatidyl choline to detergent being in the range of 1:0.5 to 1:4.0 and the weight ratio of apolipoprotein A-I to phosphatidyl choline being in the range of 1:1.5 to 1:5.0,
-- the obtained apolipoprotein A-I- phosphatidyl choline-detergent mixture is subsequently incubated at a temperature in the range of the phase change temperature ± 10 °C of the phosphatidyl choline in water,
-- the detergent is separated by means of exclusion by size or adsorption on an adsorbent, to the point where apolipoprotein A-l-phosphatidyl choline particles form with a diameter of 5 - 50 nm and a mass of 50 000 to 1 000 000 Dalton,
-- without further purification steps, a preparation is obtained which contains the reconstituted high density lipoprotein particles (rHDL) from apolipoprotein A-I and phosphatidyl choline, in which more than 95% of the apolipoprotein A-I used as well as more than 90% of the total phosphtidyl choline are bound.

2. Method for industrially producing a stable lyophilisate preparation containing reconstituted high density lipoprotein (rHDL) particles from apolipoprotein A-I and phosphatidyl choline, **characterised in that**
-- an apolipoprotein A-I phosphatidyl choline-detergent-mixture is prepared **in that** (a) an aqueous apolipoprotein A-I solution with a concentration of 1 - 40 g protein/I is mixed with (b) an aqueous phosphatidyl choline-detergent-solution, without use of organic solvents, the molar ratio phosphatidyl choline to detergent being in the range of 1:05 to 1:4.0 and the weight ratio apolipoprotein Al to phosphatidyl choline being in the range of 1:1.5 to 1: 5.0,
-- the obtained apolipoprotein Al-phosphatidyl choline-detergent-mixture is subsequently incubated at a temperature in the range of the phase change temperature ± 10 °C of the phosphatidyl choline in water,
-- the detergent is separated by means of exclusion by size or adsorption on an adsorbent, to the point where apolipoprotein A-I-phosphatidyl choline particles form with a diameter of 5 - 50 nm and a mass of 50 000 to 1 000 000 Dalton,
-- without further purification steps, a preparation is obtained which contains the reconstituted high density lipoprotein particles (rHDL) from apolipoprotein A-I and phosphatidyl choline, in which more than 95% of the apolipoprotein A-I used as well as more than 90% of the total phosphatidyl choline are bound, in the form of a liquid product, which, with a protein content of 20 ± 2 g/l and at a temperature of 20°C ± 2°C, has a turbidity of less than 40 NTU, and the product obtained is stabilised through lyophilisation in the presence of a stabiliser selected from the group of carbohydrates.

3. Method according to claim 1 or 2, **characterised in that** the separation of the detergent takes place through exclusion by size by ultrafiltration, at most 2 I of buffer per g of protein being used.

4. Method according to one of the claims 1 to 3, **characterised in that** after incubation of the lipid-apolipoprotein-detergent mixture, the detergent is bound by means of adsorption on a hydrophobic adsorbent either by batch adsorption or column method.

5. Method according to claim 4, **characterised in that** the hydrophobic adsorbent is an insoluble cross-linked polystyrene copolymer.

6. Method according to one of the claims 1 to 5, **characterised in that** the apolipoprotein A-I phosphatidyl choline-detergent solution contains phospholipids, cholesterol, cholesterol esters, fatty acids and/or triglycerides.

7. Method according to one of the claims 1 to 6, **characterised in that** used as apolipoprotein A-I is a fraction from human plasma enriched in apolipoprotein A-I which has been treated with at least one virus inactivation step by means of incubation in an aqueous solution of a chaotropic substance and a subsequent change of buffer in a solution with an ionic strength of less than 50 mmol/l, after which more than 70% of the lipoproteins are in monomeric form.

8. Method according to one of the claims 1 to 7, **characterised in that** the phosphatidyl choline is synthetic phosphatidyl choline.

9. Method according to one of the claims 1 to 8, **characterised in that** the phosphatidyl choline is a natural phosphatidyl choline, preferably a phosphatidyl choline extracted from soybeans or eggs.

10. Method according to claim 9, **characterised in that** soy phosphatidyl choline is used, and the incubation of the apolipoprotein A-I-phosphatidyl choline-detergent mixture takes place at a temperature of 0- 15 °C.

11. Method according to one of the claims 1 to 10, **characterised in that** the detergent used is selected from the class of bile acids or a salt therefrom.

12. Method according to claim 11, **characterised in that** the detergent is cholic acid - sodium salt or sodium - deoxycholic acid.

13. Method according to one of the claims 1 to 12, **characterised in that** the aqueous apolipoprotein A-I solution contains recombinant apolipoproteins, apolipoproteins from an enriched fraction of apolipoprotein A-I from human plasma, fragments of apolipoproteins or suitable, natural, synthetic or recombinant polypeptides with amphipathic properties.

14. Method according to one of the claims 1 to 13, **characterised in that** after incubation of the apolipoprotein A-I-phosphatidyl choline- detergent mixture, the content of detergent is reduced by means of dialysis or diafiltration to a concentration of less than 0.5 g detergent per g protein.

15. Method according to one of the claims 1 to 14, **characterised in that** before or after the separation of the detergent, the protein concentration is increased to 10 - 50 g/l by means of diafiltration.

16. Method according to one of the claims 1 to 15, **characterised in that** the aqueous apolipoprotein A-I solution or the lipid-detergent solution is buffered at a pH in the range of pH 6 to pH 9.

17. Method according to one of the claims 1 to 16, **characterised in that** the aqueous apolipoprotein solution or the phosphatidyl choline-detergent-solution is buffered at a pH in the range of pH 7.5 to pH 8.5.

18. Method according to one of the claims 2 to 17, **characterised in that** the product is adjusted to a protein concentration of 5 - 50 g/l and the lyophilisation of the solution to stabilise the product takes place in the presence of 5-15% of a disaccharide such as sucrose or 2-10% of a monosaccharide such as mannitol.

## Revendications

1. Procédé de préparation industrielle d'une préparation qui contient des particules reconstituées de lipoprotéines haute densité (rHDL), constituées d'apolipoprotéine A-I et de phosphatidylcholine, et qui pour une teneur en protéines de 20 ± 2 g/l et à une température de 20°C ± 2°C présente une turbidité inférieure à 40 NTU, **caractérisé en ce que**
- on prépare un mélange apolipoprotéine A-I-phosphatidylcholine-détergent en mélangeant (a) une solution aqueuse d'apolipoprotéine A-I ayant une concentration de 1 à 40 g de protéine/I à (b) une solution aqueuse de phosphatidylcholine-détergent, sans utilisation de solvants organiques, le rapport en moles de la phosphatidylcholine au détergent étant compris dans la plage de 1:0,5 à 1:4,0, et le rapport en poids de l'apolipoprotéine A-I à la phosphatidylcholine étant compris dans la plage de 1:1,5 à 1:5,0,
- puis, à une température s'écartant de moins de 10°C, dans un sens ou dans l'autre, de la température de conversion des phases de la phosphatidylcholine, on incube dans l'eau le mélange apolipoprotéine A-I-phosphatidylcholine-détergent obtenu,
- par exclusion de taille ou adsorption par un adsorbant, on sépare le détergent jusqu'à ce que se forment des particules d'apolipoprotéine A-I-phosphatidylcholine ayant un diamètre de 5 à 50 nm pour une masse de 50 000 à 1 000 000 daltons,
- où, sans autre étape de purification, on obtient une préparation qui contient des particules reconstituées de lipoprotéine haute densité (rHDL) constituées d'apolipoprotéine A-I et de phosphatidylcholine, préparation dans laquelle sont liés tant plus de 95 % de l'apolipoprotéine A-I utilisée que plus de 90 % de la totalité de la phosphatidylcholine.

2. Procédé de préparation industrielle d'une préparation stable d'un lyophilisat qui contient des particules reconstituées de lipoprotéine haute densité (rHDL), constituées d'apolipoprotéine A-I et de phosphatidylcholine, **caractérisé en ce que**
- on prépare un mélange apolipoprotéine A-I-phosphatidylcholine-détergent en mélangeant (a) une solution aqueuse d'apolipoprotéine A-I ayant une concentration de 1 à 40 g de protéine/I à (b) une solution aqueuse de phosphatidylcholine-détergent, sans utilisation de solvants organiques, le rapport en moles de la phosphatidylcholine au détergent étant compris dans la plage de 1:0,5 à 1:4,0, et le rapport en poids de l'apolipoprotéine A-I à la phosphatidylcholine étant compris dans la plage de 1:1,5 à 1:5,0,
- puis, à une température s'écartant de moins de 10°C, dans un sens ou dans l'autre, de la température de conversion des phases de la phosphatidylcholine, on incube dans l'eau le mélange apolipoprotéine A-I-phosphatidylcholine-détergent obtenu,
- par exclusion de taille ou adsorption par un adsorbant, on sépare le détergent jusqu'à ce que se forment des particules d'apolipoprotéine A-I-phosphatidylcholine ayant un diamètre de 5 à 50 nm pour une masse de 50 000 à 1 000 000 daltons,
- où, sans autre étape de purification, on obtient une préparation qui contient des particules reconstituées de lipoprotéine haute densité (rHDL) constituées d'apolipoprotéine A-I et de phosphatidylcholine, préparation dans laquelle sont liés tant plus de 95 % de l'apolipoprotéine A-I utilisée que plus de 90 % de la totalité de la phosphatidylcholine, sous forme d'un produit liquide qui, pour une teneur en protéine de 20 ± 2 g/l et à une température de 20°C ± 2°C, présente une turbidité inférieure à 40 NTU, et le produit obtenu est stabilisé par lyophilisation en présence d'un stabilisant choisi dans l'ensemble des hydrates de carbone.

3. Procédé selon la revendication 1 ou 2, **caractérisé en ce que** la séparation du détergent par exclusion de taille s'effectue par ultrafiltration, auquel cas on utilise au plus 2 I de tampon par gramme de protéine.

4. Procédé selon l'une des revendications 1 à 3, **caractérisé en ce que**, après incubation du mélange lipide-apolipoprotéine-détergent, on lie le détergent par adsorption par un adsorbant hydrophobe, par adsorption discontinue ou par un procédé sur colonne.

5. Procédé selon la revendication 4, **caractérisé en ce que** l'adsorbant hydrophobe est un copolymère du polystyrène réticulé insoluble.

6. Procédé selon l'une des revendications 1 à 5, **caractérisé en ce que** la solution d'apolipoprotéine A-I-phosphatylcholine-détergent contient des phospholipides, du cholestérol, des esters du cholestérol, des acides gras et/ou des triglycérides.

7. Procédé selon l'une des revendications 1 à 6, **caractérisé en ce qu'**on utilise en tant qu'apolipoprotéine A-I une fraction enrichie en apolipoprotéine A-I provenant du plasma humain, fraction qui a été traitée par au moins une étape d'inactivation de virus, à l'aide d'une incubation dans une solution aqueuse d'une substance chaotrope, puis retamponnage dans une solution ayant une force ionique inférieure à 50 mmoles/l, ce après quoi on est en présence de plus de 70 % de la lipoprotéine sous forme monomère.

8. Procédé selon l'une des revendications 1 à 7, **caractérisé en ce que** la phosphatidylcholine est une phosphatidylcholine synthétique.

9. Procédé selon l'une des revendications 1 à 8, **caractérisé en ce que** la phosphatidylcholine est une phosphtatidylcholine naturelle, de préférence une phosphatidylcholine extraite du soja ou de l'oeuf.

10. Procédé selon la revendication 9, **caractérisé en ce qu'**on utilise de la phosphatidylcholine du soja, et que l'incubation du mélange apolipoprotéine A-l-phosphatidylcholine-détergent s'effectue à une température de 0 à 15°C.

11. Procédé selon l'une des revendications 1 à 10, **caractérisé en ce que** le détergent utilisé est choisi dans l'ensemble des acides biliaires ou les sels de ces derniers.

12. Procédé selon la revendication 11, **caractérisé en ce que** le détergent est le sel de sodium de l'acide cholique ou le désoxycholate de sodium.

13. Procédé selon l'une des revendications 1 à 12, **caractérisé en ce que** la solution aqueuse d'apolipoprotéine A-I contient des apolipoprotéines recombinantes, des apolipoprotéines provenant d'une fraction enrichie d'apolipoprotéine A-I provenant du plasma humain, des fragments d'apolipoprotéines ou des polypeptides naturels, synthétiques ou recombinants appropriés, ayant des propriétés amphiphatiques.

14. Procédé selon l'une des revendications 1 à 13, **caractérisé en ce que**, après incubation du mélange apolipoprotéine A-I-phosphatidylcholine-détergent, on abaisse la teneur en le détergent, par dialyse ou diafiltration, à une concentration inférieure à 0,5 g de détergent par gramme de protéine.

15. Procédé selon l'une des revendications 1 à 14, **caractérisé en ce que**, avant ou après séparation du détergent, on augmente la concentration de la protéine à 10-50 g/l par diafiltration.

16. Procédé selon l'une des revendications 1 à 15, **caractérisé en ce que** la solution aqueuse d'apolipoprotéine A-I ou la solution lipide-détergent est tamponnée à un pH compris entre pH 6 et pH 9.

17. Procédé selon l'une des revendications 1 à 16, **caractérisé en ce que** la solution aqueuse d'apolipoprotéine ou la solution de phosphatidylcholine-détergent est tamponnée à un pH compris entre pH 7,5 et 8,5.

18. Procédé selon l'une des revendications 2 à 17, **caractérisé en ce que** le produit est ajusté à une concentration de protéine de 5 à 50 g/l, et la lyophilisation de la solution, pour stabiliser le produit, est réalisée en présence de 5 à 15 % d'un disaccharide, tel que le saccharose, ou de 2 à 10 % d'un monosaccharide, tel que le mannitol.
